(19) Europäisches Patentamt
European Patent Office
Office européen des brevets

(11) Publication number: **0 310 061 A1**

# (12) EUROPEAN PATENT APPLICATION

(21) Application number: 88116061.8

(51) Int. Cl.[4]: C07D 413/06 , A61K 31/42

(22) Date of filing: 29.09.88

Claims for the following Contracting State: ES.

(30) Priority: 02.10.87 US 104692
02.10.87 US 104747
02.10.87 US 104701

(43) Date of publication of application:
05.04.89 Bulletin 89/14

(84) Designated Contracting States:
AT BE CH DE ES FR GB IT LI LU NL SE

(71) Applicant: PENNWALT CORPORATION
Pennwalt Building Three Parkway
Philadelphia Pennsylvania 19102(US)

(72) Inventor: Georgiev, Vassil Stefanov
P.O. Box 1032
Penfield New York 14526(US)
Inventor: Mullen, George Byron
1385 Jenks Road
Avon New York 14414(US)

(74) Representative: Kraus, Walter, Dr. et al
Patentanwälte Kraus, Weisert & Partner
Thomas-Wimmer-Ring 15
D-8000 München 22(DE)

## (54) 5-Mono or Disubstituted-3-(phenyl or 2-naphthalenyl)-3-[(1H-imidazol-1-ylmethyl) or (1H-1,2,4-triazol-1-ylmethyl)]-2-(methyl or benzyl)isoxazolidines.

(57) 5-Mono or Disubstituted-3-(phenyl or 2-naphthalenyl)-3-[(1H-imidazol-1-ylmethyl) or (1H-1,2,4-triazol-1-ylmethyl)]-2-(methyl or benzyl)isoxazolidine compounds are disclosed which are useful as antifungal agents.

EP 0 310 061 A1

# 5-Mono or Disubstituted-3-(phenyl or 2-naphthalenyl)-3-[(1H-imidazol-1-ylmethyl) or (1H-1,2,4-triazol-1-ylmethyl)]-2-(methyl or benzyl)isoxazolidines

(IR 30101) (IRs 3010, 3011 and 3012 combined)

## Background of the Invention

This invention relates generally to substituted 2-(methyl or benzyl)isoxazolidines and more specifically to 5-mono or disubstituted-3-(phenyl or 2-naphthalenyl)-3-[(1H-imidazol-1-ylmethyl) or (1H-1,2,4-triazol-1-ylmethyl)]-2-(methyl or benzyl)isoxazolidines and related derivatives which are useful as antifungal agents.

## Brief Summary of the Invention

In accordance with this invention there are provided compounds of the formula:

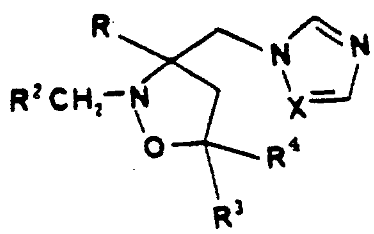

and the pharmaceutically acceptable acid addition salts thereof, in the form of their enantiomers or mixtures of their enantiomers including diastereomeric pairs of such enantiomers,
wherein X is selected from CH or N and wherein R, $R^2$, $R^3$ and $R^4$ are selected from the following combinations;

(a) R is,

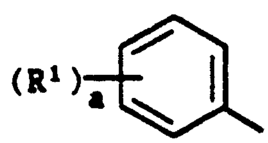

wherein a = 1 or 2, and
$R^1$ is selected from hydrogen, halogen, lower alkyl, lower alkoxy groups and combinations thereof, provided that the ortho position is hydrogen,
$R^2$ is,

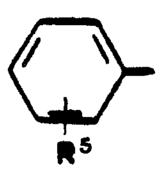

wherein $R^5$ is selected from hydrogen and one or more halogen, lower alkyl and lower alkoxy groups and combinations thereof,
$R^3$ is selected from phenyl, substituted phenyl, styryl, substituted styryl, substituted phenoxymethyl and

substituted phenylthiomethyl groups wherein the substituents at the phenyl rings are selected from one or more halogen, lower alkoxy and lower alkyl groups and combinations thereof, and
$R^4$ is H,

(b) R is defined as in (a) above,
$R^2$ is H, and
$R^3$ is selected from lower alkyl, phenyl, substituted phenyl and alkoxycarbonyl groups and
$R^4$ is selected from lower alkyl, phenyl and substituted phenyl groups, wherein the phenyl substituents are selected from halogen, lower alkyl, and lower alkoxy groups and combinations thereof, and

(c) R is naphthyl,
$R^2$ and $R^4$ are H, and
$R^3$ is selected from phenyl, substituted phenyl, phenoxymethyl, substituted phenoxymethyl, (phenylthio)-methyl, substituted (phenylthio)methyl, styryl and $C_2$ to $C_{18}$ alkyl, wherein the substituents on the substituted phenyl rings are selected from one or more of halogen, lower alkyl, lower alkoxy groups and combinations thereof.

## Detailed Description of the Invention

The compounds of this invention are useful as antifungal agents. They have in vitro activity against yeast and systemic mycoses and dermatophytes as determined by broth and agar testing techniques [McGinnis, M.R., Laboratory Handbook of Medical Mycology, Academic Press, New York, New York (1980)]. The compounds have good to moderate inhibitory activity against a variety or organisms including Trichophyton mentagrophytes, Trichophyton rubrum, Trichophyton tonsurans, Trichophyton schoenleinii, Epidermophyton floccosum, Microsporum canis, Aspergillus fumigatus, Candida albicans and Candida stellatoidea (minimum inhibitory concentration, MIC, of 0.2 to 70 µg/ml).

Because of the antifungal activity of the compounds of the invention they can be used, for example, in suitable liquid, semi-solid or solid carriers in the form of solutions, emulsions, suspensions, dispersions, ointments, aerosols, soaps, detergents, and powders in amounts effective to combat systemic and dermatophylic fungal infections in warm blooded animals (1 to 20 percent active ingredient).

In the definition of the compounds of the invention by halogen is meant chlorine, bromine, fluorine and iodine with chlorine and fluorine being preferred. By lower alkyl is meant alkyl groups containing one to four (1-4) carbons. By lower alkoxy is meant such groups containing one to six (1-6) carbons. Alkyl groups with three or more carbons can have a branched or unbranched chain. Preferably the substituted phenyl groups have one or two substituents.

The compounds of the invention are obtained as a mixture of cis- and trans-diastereomers due to the presence of two asymmetric carbon atoms in the isoxazolidine ring. The diastereomeric mixture is conveniently separated by flash chromatography on silica gel using halogenated hydrocarbons (preferably dichloromethane and chloroform), alkanols (preferably methanol and ethanol), ethyl acetate and such, as eluents. The eluents may be used alone or in combinations such as the ones comprised of 95-99% halogenated hydrocarbon and 1-5% alkanol by volume. The stereochemistry of the two asymmetric carbon atoms in the isoxazolidine ring may be determined by conventional methods that include x-ray crystallography, nuclear magnetic resonance spectroscopy, circular dichroism and optical rotatory dispersion. Both the cis- and trans-diastereoisomers are resolvable into their optical enantiomers with (+)- and (-)-optical rotations by standard techniques such as fractional recrystallization of the diastereomeric salts with optically active organic acids such (+)- and (-)-tartaric acid, (+)- and (-)-dibenzoyl-tartaric acid and the like.

The 2-benzyl (or 2-phenylmethyl) compounds of the invention can be prepared as illustrated in the following diagram. The synthesis of the nitrone precursors 3 is accomplished by reacting an appropriately substituted 2-imidazolylacetophenone 1 with substituted N-benzylhydroxylamine 2. Subsequent reaction of the nitrone 3 with an appropriate 1-alkene derivative 4 provides a diastereomeric mixture of the desired cis- and trans-5-substituted-3-phenyl-3-(1H-imidazol-1-ylmethyl)-2-benzylisoxazolidine derivative 5.

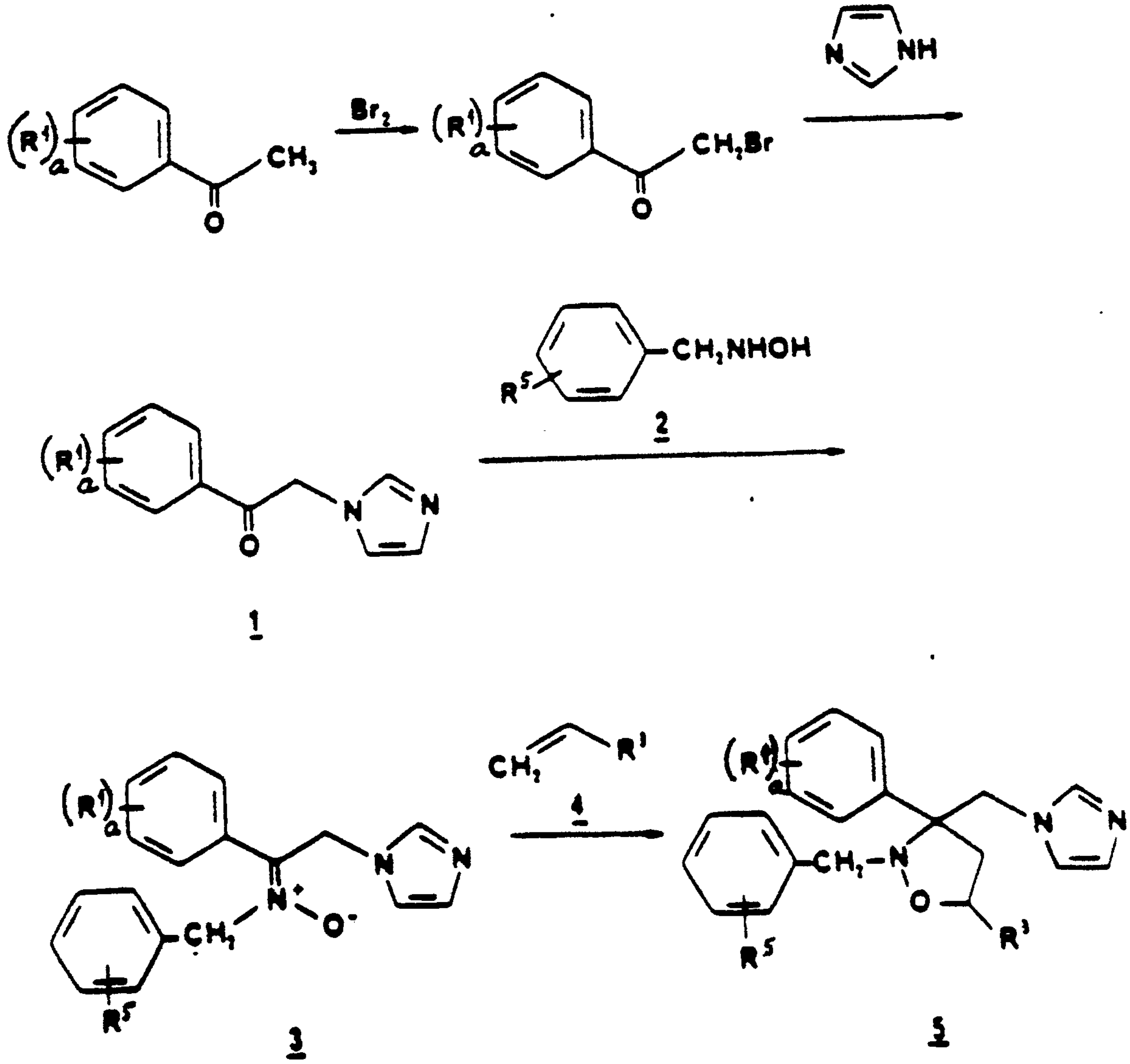


1

3

5

Similarly by using 2-(1H-1,2,4,-triazol-1-yl)acetophenone, the corresponding 3-(1H-1,2,4-triazol-1-ylmethyl) isoxazolidine can be prepared.

The 5,5-disubstituted-3-phenyl-3-[1H-imidazol-1-ylmethyl)-2-methylisoxazolidine derivatives 8 of this invention are prepared according to the following scheme with nitrone 6 being reacted with an appropriate 1-alkene derivative 7 to provide a diastereomeric mixture of the desired product 8.

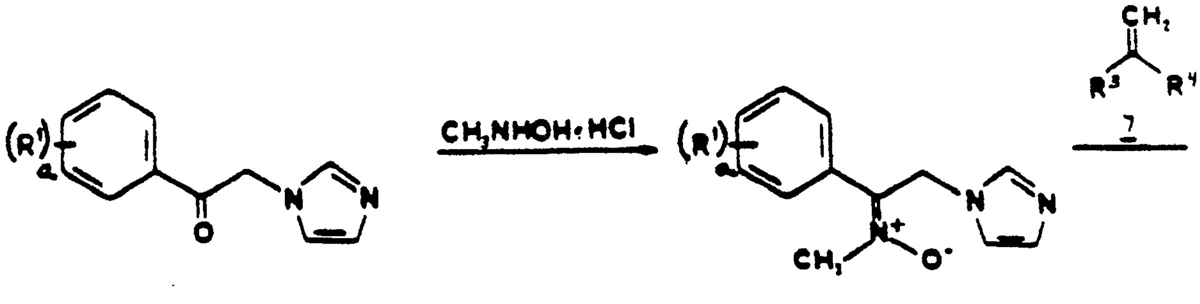


6

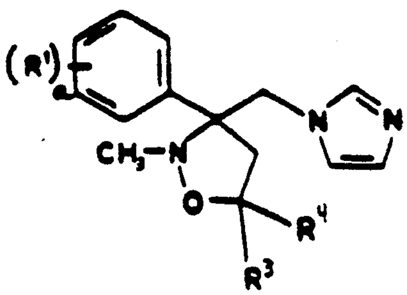

8

As above, the corresponding 3-(1H-1,2,4-triazol-1-ylmethyl)isoxazolidine derivatives are prepared starting with an appropriate 2-(1H-1,2,4-triazol-1-yl)acetophenone to form the corresponding nitrone.

The 5-substituted-3-(2-naphthalenyl)-3-[(1H-imidazol-1-ylmethyl) or (1H-1,2,4-triazol-1-ylmethyl)]-2-methylisoxazolidine derivatives of this invention are prepared starting with, for example, the reaction of 2-imidazolyl [or 2-(1H-1,2,4-triazol-1-yl)]acetonaphthone with N-methylhydroxylamine hydrochloride to furnish (in the case of the imidazolyl) the corresponding nitrone derivative 10. Subsequent reaction of compound 10 with 1-alkene derivatives provides diastereomeric mixtures of the desired *cis*- and *trans*-isoxazolidine compounds 11.

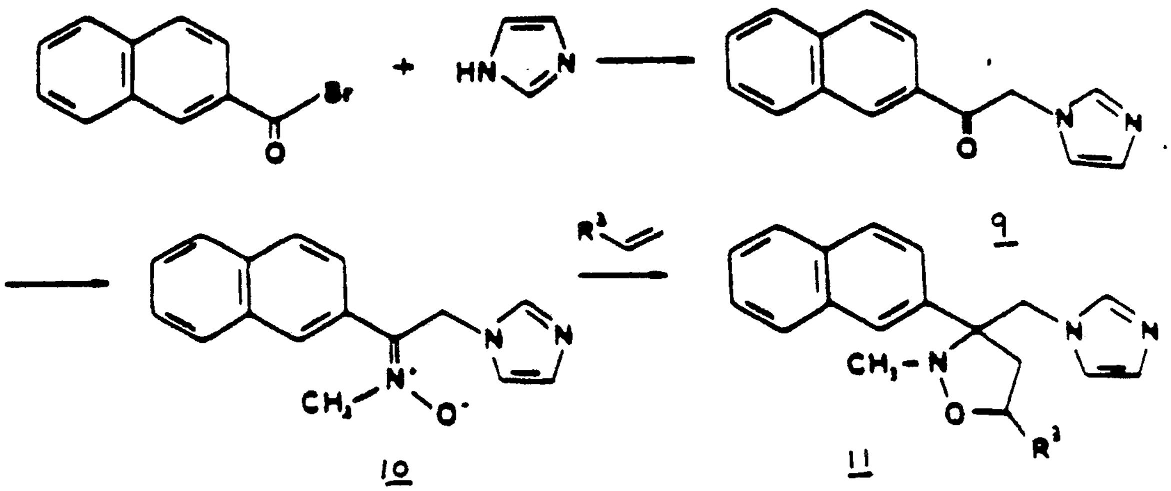


10 11

The compounds of this invention are all basic and thus can form salts with pharmaceutically acceptable inorganic and organic acids such as, for example, acetic acid, maleic acid, malic acid, fumaric acid, succinic

acid, succinamic acid, tartaric acid, citric acid, lactic acid, hydrochloric acid, hydrobromic acid, hydroiodic acid, nitric acid, sulfuric acid and phosphoric acid.

The preparation of the compounds of this invention is further illustrated by the following synthesis of intermediates and in the Examples.

## Preparation of Hydroxylamine Intermediates

The hydroxylamines 2 were prepared from their corresponding oximes by the method of Kawase and Kikugawa [J. Chem. Soc., Perkin I, 643-645 (1979)]:

(a) N-[(3-Chlorophenyl)methyl]hydroxylamine hydrochloride (2, $R^5$ = 3-Cl), m.p. 161-169 °C (ethanol),

(b) N-[(4-Fluorophenyl)methyl]hydroxylamine hydrochloride (2, $R^5$ = 4-F), m.p. 70-76 °C (ether),

(c) N-[(4-Methoxyphenyl)methyl]hydroxylamine (2, $R^5$ = 4-$OCH_3$), m.p. 65-70 °C (ethyl acetate/hexane, 1:1 by volume).

## Example 1

3-)4-Fluorophenyl)-3-(1H-imidazol-1-ylmethyl)-5-phenyl-2-(phenylmethyl)isoxazolidine (5, $R^1$ = 4-F, $R^5$ = H, $R^3$ = $C_6H_5$

A suspension of 2.00 g (0.0098 mol) of 1-(4-fluorophenyl)-2-(1H-imidazol-1-yl)ethanone (1, $R^1$ = 4-F), 1.30 g (0.011 mol) of N-(phenylmethyl)hydroxylamine (2, $R^5$ = H), 0.82 g (0.010 mol) of sodium acetate, and 0.60 ml (0.010 mol) of acetic acid in 25 ml of ethanol is heated to reflux under a nitrogen atmosphere and stirred for 24 hours. Upon cooling to ambient temperature, the suspension is poured into 50 ml of water, neutralized with sodium bicarbonate and extracted with chloroform (2 x 50 ml). The combined organic extract is dried over anhydrous magnesium sulfate, concentrated in vacuo, and flash-chromatographed on neutral silica gel using a 9:1 by volume mixture of ethyl acetate and methanol as the eluent, to give 1.63 g (54%) of 1-(4-fluorophenyl)-2-(1H-imidazol-1-yl)-N-(phenylmethyl) ethanimine N-oxide (3, $R^1$ = 4-F, $R^5$ = H) as a light yellow oil. This oil (0.0053 mol) is dissolved in 50 ml of toluene, 1.5 ml (0.013 mol) of styrene (4, $R^3$ = $C_6H_5$) is added, and the solution is refluxed under a nitrogen atmosphere for 28 hours, cooled to ambient temperature and concentrated in vacuo. The resulting cis- and trans-diastereomeric mixture of compound 5 ($R^1$ = 4-F, $R^5$ = H, $R^3$ = $C_6H_5$) is flash-chromatographed on neutral silica gel using a 98:2 by volume mixture of chloroform and methanol as the eluent.

Isomer A (0.98 g, 45%) has a melting point of 162-164 °C (ethyl acetate). Anal. Calcd. for $C_{26}H_{24}FN_3O$: C, 75.52; H, 5.85; F, 4.59; N, 10.16. Found: C, 75.50; H, 6.04; F, 4.74; N, 10.05.

## Example 2

3-(4-Chlorophenyl)-3-(1H-imidazol-1-ylmethyl)-2-[(4-methoxyphenyl)methyl]-5-phenylisoxazolidine (5, $R^1$ = 4-Cl, $R^5$ = 4-$OCH_3$, $R^3$ = $C_6H_5$)

Compound 5 ($R^1$ = 4-Cl, $R^5$ = 4-$OCH_3$, $R^3$ = $C_6H_5$) is prepared by a method similar to that described in Example 1 by reacting 8.06 g (0.0365 mol) of 1-(4-chlorophenyl)-2-(1H-imidazol-1-yl)ethanone (1, $R^1$ = 4-Cl) with 8.39 g (0.055 mol) of N-[(4-methoxyphenyl)methyl]hydroxylamine (2, $R^5$ = 4-$OCH_3$) to give 4.42 g (34%) of 1-(4-chlorophenyl)-2-(1H-imidazol-1-yl)-N-[(4-methoxyphenyl)methyl]ethanimine N-oxide (3, $R^1$ = 4-Cl, $R^5$ = 4-$OCH_3$) as a light yellow oil, followed by reaction of 3 ($R^1$ = 4-Cl, $R^5$ = 4-$OCH_3$) (4.42 g, 0.0124 mol) with 1.90 g (0.018 mol) of styrene (4, $R^3$ = $C_6H_5$). The resulting cis- and trans-diastereomeric mixture of compound 5 ($R^1$ = 4-Cl, $R^5$ = 4-$OCH_3$, $R^3$ = $C_6H_5$) is flash-chromatographed on neutral silica gel using a 95:5 by volume mixture of ethyl acetate and methanol (saturated with ammonia) as the eluent.

Isomer A (2.61 g, 46%) has a melting point of 162-167° C (ethyl acetate-hexane, 1:1 by volume). Anal. Calcd. for $C_{27}H_{26}ClN_3O_2$: C, 70,50; H, 5.70; Cl, 7.71; N, 9.14. Found: C, 70.40; H, 5.80; Cl, 8.13; N, 9.14.

## Example 3

5-[(4-Chlorophenoxy)methyl]-3-(1H-imidazol-1-ylmethyl)-3-phenyl-2-(phenylmethyl)isoxazolidine (5, $R^1$ = $R^5$ = H, $R^3$ = $CH_2OC_6H_4Cl$-4)

Compound 5 ($R^1$ = $R^5$ = H, $R^3$ = $CH_2OC_6H_4Cl$-4) is prepared by a method similar to that described in Example 1 by reacting 3.72 g (0.020 mol) of 2-(1H-imidazol-1-yl)-1-phenylethanone (1, $R^1$ = H) with 2.95 g (0.024 mol) of N-(phenylmethyl)hydroxylamine (2, $R^5$ = H) to give 2-(1H-imidazol-1-yl)-1-phenyl-N-(phenylmethyl)ethanimine N-oxide (3, $R^1$ = $R^5$ = H), followed by reaction of 3 ($R^1$ = $R^5$ = H) with 3.59 g (0.021 mol) of allyl 4-chlorophenyl ether (3, $R^3$ = $CH_2OC_6H_4Cl$-4). The resulting cis- and trans-diastereomeric mixture of compound 5 ($R^1$ = $R^5$ = H, $R^3$ = $CH_2OC_6H_4Cl$-4) is flash-chromatographed on neutral silica gel using a 98:2 by volume mixture of chloroform and methanol as the eluent.

Isomer A (2.55 g, 27.7% overall) has a melting point of 128-130° C (ethyl acetate-hexane, 1:1). Anal. Calcd. for $C_{27}H_{26}ClN_3O_2$: C, 70.50; H, 5.70; Cl, 7.71; N, 9.14. Found: C, 70.48; H, 5.82; Cl, 7.85; N, 9.06.

Isomer B (0.040 g, 4.3% overall) has a melting point of 110-113° C (ethyl acetate-hexane, 1:1). Anal. Calcd. for $C_{27}H_{26}ClN_3O_2$: C, 70.50; H, 5.70; Cl, 7.71; N, 9.14. Found : C, 70.16; H, 5.56; Cl, 7.88, N, 9.12.

## Example 4

5-[(4-Chlorophenoxy)methyl]-2-[(3-chlorophenyl)methyl]-3-(1H-imidazol-1-yl-methyl)-3-phenylisoxazolidine - (5, $R^1$ = H, $R^5$ = 3-Cl, $R^3$ = $CH_2OC_6H_4Cl$-4)

Compound 5 ($R^1$ = H, $R^5$ = 3-Cl, $R^3$ = $CH_2OC_6H_4Cl$-4) is prepared by a method similar to that described in Example 1 by reacting 5.20 g (0.028 mol) of 2-(1H-imidazol-1-yl)-1-phenylethanone (1, $R^1$ = H) with 8.20 g (0.042 mol) of N-[(3-chlorophenyl)methyl]hydroxylamine hydrochloride (2, $R^5$ = 3-Cl) to give 5.39 g (59%) of N-[(3-chlorophenyl)methyl]-2-(1H-imidazol-1-yl)-1-phenylethanimine N-oxide (3, $R^1$ = H, $R^5$ = 3-Cl), followed by reaction of 3 ($R^1$ = H, $R^5$ = 3-Cl) (5.39 g, 0.0165 mol) with 4.20 g (0.0248 mol) of allyl 4-chlorophenyl ether (4, $R^3$ = $CH_2OC_6H_4Cl$-4). The resulting cis- and trans-diastereomeric mixture of compound 5 ($R^1$ = H, $R^5$ = 3-Cl, $R^3$ = $CH_2OC_6H_4Cl$-4) is flash-chromatographed on neutral silica gel using a 98:2 by volume mixture of chloroform and methanol as the eluent.

Isomer A (2.0 g, 2.45%) has a melting point of 103-107° C (ethyl acetate-hexane, 1:1). Anal. Calcd. for $C_{27}H_{25}ClN_3O_2$: C, 65.59; H, 5.10; Cl, 14.34; N, 8.50. Found: C, 65.47; H, 5.15; Cl, 14.50; N, 8.48.

## Example 5

2-[(4-Fluorophenyl)methyl]-3-(1H-imidazol-1-ylmethyl)-5-[[(4-methylphenyl)thio]methyl]-3-phenylisoxazolidine (5, $R^1$ = H, $R^5$ = 4-F, $R^3$ = $CH_2SC_6H_4CH_3$-4)

Compound 5 ($R^1$ = H, $R^5$ = 4-F, $R^3$ = $CH_2SC_6H_4CH_3$-4) is prepared by a method similar to that described in Example 1 by reacting 3.20 g (0.0173 mol) of 2-(1H-imidazol-1-yl)-1-phenylethanone (1, $R^1$ = H) with 3.66 g (0.0259 mol) of N-[(4-fluorophenyl)methyl]hydroxylamine (2, $R^5$ = 4-F) to give 2.44 g (45.6%) of N-[(4-fluorophenyl)methyl]-2-(1H-imidazol-1-yl)-1-phenylethanimine N-oxide (3, $R^1$ = H, $R^5$ = 4-F), followed by reaction of 3 ($R^1$ = H, $R^5$ = 4-F) (1.49 g, 0.0048 mol) with 1.20 g (0.007 mol) of allyl 4-methylphenyl sulfide (4, $R^3$ = $CH_2SC_6H_4CH_3$-4). The resulting cis- and trans-diastereomeric mixture of compound 5 ($R^1$ = H, $R^5$ = 4-F, $R^3$ = $CH_2SC_6H_4CH_3$-4) is flash-chromatographed on neutral silica gel using a 98:2 by volume mixture of chloroform and methanol as the eluent.

Isomer A (1.26 g, 55%) has a melting point of 168-172 °C (ethyl acetate) as its monohydrochloride salt. Anal. Calcd. for $C_{28}H_{29}ClFN_3OS$: C, 65.93; H, 5.73; Cl, 6.95; F, 3.72; N, 8.24; S, 6.29. Found: C, 65.59; H, 5.88; Cl, 7.37; F, 3.64; N, 8.09; S, 6.62.

## Example 6

5-Substituted-3-(phenyl or substituted phenyl)-3-(1H-1,2,4-triazol-1-ylmethyl)-2-(benzyl or substituted benzyl)isoxazolidines (X = N)

By following essentially the same methods as described for Examples 1-5 and replacing 1-(phenyl or substituted phenyl)-2-(1H-imidazol-1-yl)-N-[(phenyl or substituted phenyl)methyl]ethanimine N-oxide by,

1-(phenyl or substituted phenyl)-2-(1H-1,2,4-triazol-1-yl)-N-[(phenyl or substituted phenyl)methyl]-ethanimine N-oxide,

the corresponding 5-substituted-3-(phenyl or substituted phenyl)-3-(1H-1,2,4-triazol-1-ylmethyl)-2-(benzyl or substituted benzyl)isoxazolidines can be prepared.

For example,

3-(4-fluorophenyl)-3-(1H-1,2,4-triazol-1-ylmethyl)-5-phenyl-2-(phenylmethyl)isoxazolidine,
3-(4-chlorophenyl)-3-(1H-1,2,4-triazol-1-ylmethyl)-2-[(4-methoxyphenyl)methyl]-5-phenylisoxazolidine,
5-[(4-chlorophenoxy)methyl]-3-(1H-1,2,4-triazol-1-ylmethyl)-3-phenyl-2-(phenylmethyl)isoxazolidine,
5-[(4-chlorophenoxy)methyl]-2-[(3-chlorophenyl)methyl]-3-(1H-1,2,4-triazol-1-ylmethyl)-3-phenylisoxazolidine,
2-[(4-fluorophenyl)methyl]-3-(1H-1,2,4-triazol-1-ylmethyl)-5-{[(4-methylphenyl)thio]methyl}-3-phenylisoxazolidine,
3-(4-methoxyphenyl)-3-(1H-1,2,4-triazol-1-ylmethyl)-5-phenyl-2-[(4-methylphenyl)methyl]isoxazolidine,
3-(4-chloro-3-methylphenyl)-3-(1H-1,2,4-triazol-1-ylmethyl)-5-(4-methoxyphenyl)-2-[(4-chloro-3-methylphenyl)methyl] isoxazolidine,
3-(4-fluorophenyl)-3-(1H-1,2,4-triazol-1-ylmethyl)-5-(2-trans-phenylethenyl)-2-[(4-methylphenyl)methyl]-isoxazolidine, and
3-(4-methylphenyl)-3-(1H-1,2,4-triazol-1-ylmethyl)-5-phenyl-2-[(3-chlorophenyl)methyl]isoxazolidine.

## Example 7

5-(4-Chlorophenyl)-2,5-dimethyl-3-(1H-imidazol-1-ylmethyl)-3-phenylisoxazolidine (8, $R^1$ = H, $R^3$ = $CH_3$, $R^4$ = $C_6H_4Cl$-4)

A solution of 10.79 g (0.0501 mol) of 2-(1H-imidazol-1-yl)-N-methyl-1-phenylethanimine N-oxide (6, $R^1$ = H) [which can be prepared by reacting 2-(1H-imidazol-1-yl)acetophenone (45.05 g, 0.204 mol), N-methylhydroxylamine hydrochloride (20.93 g, 0.251 mol) and sodium acetate (41.13 g, 0.502 mol) in 550 ml ethanol] and 8.72 g (0.0571 mol) of p-chloro-α-methylstyrene (7, $R^3$ = $CH_3$, $R^4$ = $C_6H_4Cl$-4) in 100 ml of toluene is heated to reflux and stirred under a nitrogen atmosphere for 70 hours. Upon cooling to ambient temperature, the reaction mixture is extracted with water (2 x 100 ml), and the organic layer is dried over anhydrous magnesium sulfate, then concentrated in vacuo. The residual dark oil, containing a cis- and trans-diastereomeric mixture of compound 8 ($R^1$ = H, $R^3$ = $CH_3$, $R^4$ = $C_6H_4Cl$-4), is flash-chromatographed on neutral silica gel using ethyl acetate as eluent. Isomer A (2.35 g, 12.7%) has a melting point of 180-182 °C (ethyl acetate). Anal. Calcd. for $C_{21}H_{22}ClN_3O$: C, 68.56; H, 6.03; Cl, 9.64; N, 11.42. Found: C, 68.42; H, 6.05; Cl, 9.75; N, 11.40.

Isomer B (2.26 g, 12.2%) has a melting point of 122-124 ° C (ethyl ether). Anal. Calcd. for $C_{21}H_{22}ClN_3O$: c, 68.56; H, 6.03; Cl, 9.64; N, 11.42. Found: C, 68.61; H, 6.17; Cl, 9.69; N, 11.28.

## Example 8

3-(1H-Imidazol-1-ylmethyl)-2-methyl-3,5,5-triphenylisoxazolidine (8, $R^1$ = H, $R^3$ = $R^4$ = $C_6H_5$)

Compound 8 ($R^1$ = H, $R^3$ = $R^4$ = $C_6H_5$) is prepared by a method similar to that described in Example 7 from 6.46 g (0.030 mol) of 2-(1H-imidazol-1-yl)-N-methyl-1-phenylethanimine N-oxide (6, $R^1$ = H) and 6.50 g (0.035 mol) of 1,1-diphenylethylene (7, $R^3$ = $R^4$ = $C_6H_5$) in 300 ml of toluene, followed by flash chromatography on neutral silica gel using a 97:3 by volume mixture of chloroform and methanol as the eluent.

Compound 8 ($R^1$ = H, $R^3$ = $R^4$= $C_6H_5$) (1.00 g, 8.4%) has a melting point of 183-184 °C (ethyl acetate). Anal. Calcd. for $C_{25}H_{25}N_3O$: C, 78.96; H, 6.37; N, 10.62. Found: C, 78.61; H, 6.44; N, 10.57.

## Example 9

Methyl 3-(4-Chlorophenyl)-2,5-dimethyl-3-(1H-imidazol-1-ylmethyl)isoxazolidine-5-carboxylate (8, $R^1$ = 4-Cl, $R^3$ = $CH_3$, $R^4$ = $CO_2CH_3$)

Compound 8 ($R^1$ = 4-Cl, $R^3$ = $CH_3$, $R^4$ = $CO_2CH_3$) is prepared by a method similar to that described in Example 7 from 7.04 g (0.0282 mol) of 1-(4-chlorophenyl)-2-(1H-imidazol-1-yl)-N-methylethanimine N-oxide (6, $R^1$ = 4-Cl) and 4.0 ml (1.30 equiv) of methyl methacrylate (7, $R^3$ = $CH_3$, $R^4$ = $CO_2CH_3$). The resulting cis- and trans- diastereomeric mixture of compound 8 ($R^1$ = 4-Cl, $R^3$ = $CH_3$, $R^4$ = $CO_2CH_3$) is flash-chromatographed on neutral silica gel using ethyl acetate as the eluent.

Isomer A (4.28 g, 43.4%) has a melting point of 119-122 ° C (ethyl acetate). Anal. Calcd. for $C_{17}H_{20}ClN_3O_3$: C, 58.37; H, 5.76; Cl, 10.13; N, 12.01. Found: C, 58,36; H, 5.74; Cl, 10.17; N, 11.98.

## Example 10

3-(Substituted phenyl)-2,5-dimethyl-3-(1H-imidazol-1-ylmethyl)-5-(substituted phenyl)isoxazolidines

By following essentially the same methods as described for Example 7 and replacing (2-(1H-imidazol-1-yl)-N-methyl-1-phenylethanimine N-oxide by,
2-(1H-imidazol-1-yl)-N-methyl-1-(4-methoxyphenyl) ethanimine N-oxide, or
2-(1H-imidazol-1-yl)-N-methyl-1-(4-methylphenyl) ethanimine N-oxide, or
2-(1H-imidazol-1-yl)-N-methyl-1-(4-chloro-3- methylphenyl)ethanimine N-oxide, or
2-(1H-imidazol-1-yl)-N-methyl-1-(4-fluorophenyl) ethanimine N-oxide, or
2-(1H-imidazole-1-yl)-methyl-1-(3,4-dichlorophenyl)ethanimine N-oxide,
and replacing p-chloro-α-methylstyrene by,
α-methylstyrene, or
4-methyl α-methylstyrene, or
4-methoxy α-methylstyrene, or
3,4-dimethoxy α-methylstyrene, or
3,4-dichloro α-methylstyrene,
the corresponding 3-(substituted phenyl)-2,5-dimethyl-3-(1H-imidazol-1-ylmethyl)-5-(phenyl or substituted phenyl) isoxazolidines can be prepared.

For example,
3-(4-methoxyphenyl)-2,5-dimethyl-3-(1H-imidazol-1-ylmethyl)-5-phenylisoxazolidine,
5-(3,4-dichlorophenyl)-2,5-dimethyl-3-(1H-imidazol-1-ylmethyl)-3-(4-methoxyphenyl)isoxazolidine,
3-(4-methylphenyl)-2,5-dimethyl-3-(1H-imidazol-1-ylmethyl)-5-(4-methoxyphenyl)isoxazolidine,
3-(4-chloro-3-methylphenyl)-2,5-dimethyl-3-(1H-imidazol-1-ylmethyl)-5-(4-methylphenyl)isoxazolidine,
5-(3,4-dimethoxylphenyl)-2,5-dimethyl-3-(1h-imidazol-1-ylmethyl)-3-(4-methylphenyl)isoxazolidine.

## Example 11

5,5-Disubstituted 3-(phenyl or substituted phenyl)-2-methyl-3-(1H-1,2,4-triazol-1-ylmethyl)isoxazolidines

By following essentially the same methods as described for examples 7-10 and substituting a 2-(1H-1,2,4-triazol-1-yl)-N-methyl-1-(phenyl or substituted phenyl)ethanimine N-oxide [prepared by reacting a 2-(1H-1,2,4-triazol-1-yl)acetophenone (or substituted acetophenone) with N-methylhydroxylamine hydrochloride] for 2-(1H-imidazol-1-yl)-N-methyl-1-(phenyl or substituted phenyl)ethanimine N-oxide, the corresponding 5,5-disubstituted-3-(phenyl or substituted phenyl)-2-methyl-3-(1H-1,2,4-triazol-1-ylmethyl)-isoxazolidines can be prepared.

For example,
5-(4-chlorophenyl)-2,5-dimethyl-3-(1H-1,2,4-triazol-1-ylmethyl)-3-phenylisoxazolidine,
3-(1H-1.2,4-triazol-1-ylmethyl)-2-methyl-3,5,5-triphenylisoxazolidine, and
methyl 3-(4-chlorophenyl)-2,5-dimethyl-3-(1H-1,2,4-triazol-1-ylmethyl)isoxazolidine-5-carboxylate.

## Preparation of Intermediates

### 2-Imidazolyl-2′-acetonaphthone (9)

To a solution of 68.32 g (1.004 mol) of imidazole dissolved in 150 ml of methanol at 0° C (ice-bath) is added dropwise a solution of 2-bromo-2′-acetonaphthone (50 g, 0.201 mol) in 100 ml of dioxane and 25 ml of ether, while keeping the temperature at 0° C. After about 3 hours at 0° C, the mixture is allowed to warm to room temperature where it is stirred for 20 hours. The mixture is then filtered and added to 500 ml of water, extracted 3 times with 500 ml of chloroform, dried over magnesium sulfate, and evaporated under reduced pressure. The brown oil that remains is crystallized with a small amount of ethyl acetate and hexane and collected by filtration. The solid is heated in ethyl acetate and filtered hot then concentrated enough to recrystallized the product as a light tan solid. Yield: 31.07 g (65%); m.p. 125-126° C. (Found: C, 76.03; H, 5.25; N, 11.81. $C_{15}H_{12}N_2O$ requires: C, 76.25; H, 5.12; N, 11.86).

### 2-(1H-Imidazol-1-yl)-N-methyl-1-(2-naphthalenyl)ethanimine N-oxide (10)

A mixture of 2-imidazolyl-2′-acetonaphthone (19.63 g, 0.083 mol), N-methylhydroxylamine-HCl(12.50 g, 0.150 mol), and sodium acetate (12.30 g, 0.150 mol), in 500 ml of absolute ethanol is stirred under nitrogen for 72 hours at 25° C. The mixture was filtered, added to water, extracted with chloroform, dried over magnesium sulfate, and evaporated to dryness. The oil that remained is crystallized with a small amount of ethyl acetate and ether. Yield: 14.93 g (68%); m.p. 112-114° C. (Found: C,72.14; H, 5.79; N, 15.74. $C_{16}H_{15}N_3O$ requires: C, 72.43; H, 5.70; N, 15.84).

## Example 12

### 3-(1H-Imidazol-1-ylmethyl)-2-methyl-3-(2-naphthalenyl)-5-(phenoxymethyl)isoxazolidine (11a) ($R^3$ = $CH_2OC_6H_5$)

A mixture of compound 10 (9.0 g, 0.034 mol) and phenyl allyl ether (6.98 ml, 0.051 mol) in 200 ml of toluene is refluxed for 48 hours, cooled and evaporated under vacuum. The semisolid that remained (7.30 g, 54%) is crystallized from ether and recrystallized from ethyl acetate to give tan crystals. Yield: 2.4 g (18%); m.p. 176-178° C. (Found: C, 74.96; H, 6.41; N, 10.40. $C_{25}H_{25}N_3O_2$ requires: C, 75.16; H, 6.31; N, 10.52).

Example 13

The following derivatives are prepared by a procedure similar to that described for derivatives 11a:

5-[(4-Chlorophenoxy)methyl]-3-(1H-imidazol-1-ylmethyl)-2- methyl-3-(2-naphthalenyl)isoxazolidine (11b) ($R^3$ = $CH_2OC_6H_4Cl$-p)

Derivative 11b is prepared from compound 10 and p-chlorophenyl allyl ether, and is recrystallized from ethyl acetate/hexane. Yield: 31%; m.p. 163-165° C. (Found: C, 69.15; H, 5.73; N, 9.60; Cl, 8.08. $C_{25}H_{24}N_3O_2Cl$ requires: C, 69.20; H, 5.57; N, 9.68; Cl, 8.17).

3-(1H-Imidazol-1-ylmethyl)-2-methyl-3-(2-naphthalenyl)-5-phenylisoxazolidine (11c) ($R^3$ = phenyl)

Derivative 11c is prepared from compound 10 and styrene and is recrystallized from ethyl acetate. Yield: 31%; m.p. 170-172° C. (Found: C, 77.95; H, 6.59; N, 11.31. $C_{24}H_{23}N_3O$ requires: C, 78.02; H. 6.27; N, 11.37).

5-(4-Chlorophenyl)-3-(1H-imidazol-1-ylmethyl)-2-methyl-3-(2-naphthalenyl)isoxazolidine (11d) ($R^3$ = $C_6H_4Cl$-p)

Derivative 11d is prepared from compound 10 and p-chlorostyrene and is recrystallized from ethyl acetate. Yield: 38%; m.p. 195-198° C. (Found: C, 71.16: H, 5.57; N, 10.32; Cl, 8.90 $C_{24}H_{22}N_3OCl$ requires: C, 71.37; H, 5.49; N, 10.40; Cl, 8.78.

5-Hexyl-3-(1H-imidazol-1-ylmethyl)-2-methyl-3-(2-naphthalenyl) isoxazolidine (11e) [$R^3$ = $(CH_2)_5CH_3$]

Derivative 11e is prepared from compound 10 and 1-octene. Purification of 11e is done by flash chromatography on silica gel with eluent comprised of 98% chloroform and 2% ethanol by volume. The compound is crystallized with ether/hexane. Yield: 12%; m.p. 92-94° C. (Found: C, 75.98; H, 8.17; N, 10.96; $C_{24}H_{31}N_3O$ requires: C, 76.36; H, 8.28; N, 11.13).

Example 14

3-(1H-Imidazol-1-ylmethyl)-5-[(4-methoxyphenoxy)methyl]-2-methyl-3-(2-naphthalenyl)isoxazolidine (11f) ($R^3$ = $CH_2OC_6H_4OCH_3$-p)

A mixture of compound 10 (13.0 g, 0.049 mol) and p-methoxyphenyl allyl ether (12.07 g, 0.073 mol) in 400 ml of toluene is refluxed for 48 hours, cooled, and the solvent removed *in vacuo* leaving a dark oil. Chloroform is added to the oil and carbon is added to decolorize the solution which is filtered through a bed of celite. The chloroform is evaporated and the oil that remained is purified by flash chromatography on silica gel (eluent comprised of 98% $CHCl_3$ and 2% MeOH). The resulting tan solid (5.56 g, 26%) is recrystallized from ethyl acetate, m.p. 143-145° C (Found: C,72.58; H, 6.46; N, 9.66. $C_{26}H_{27}N_3O_3$ requires: C, 72.71; H, 6.34; N, 9.78).

Example 15

3-(1H-Imidazol-1-ylmethyl)-2-methyl-3-(2-naphthalenyl)-5-(2-trans-phenylethenyl)isoxazolidine (11g) ($R^3$ =

$CH=CHC_6H_5$)

A mixture of compound 10 (11.02 g, 0.042 mol) and trans-1-phenyl-1,3-butadiene (8.0 g, 0.062 mol) in 500 ml of toluene is heated to 85°C and held there for 72 hours, cooled and evaporated to dryness. The oil that remained is purified by flash chromatography on silica gel (eluent comprised of 98% $CHCl_3$ and 2% MeOH by volume). The resulting semisolid is dissolved in chloroform, decolorized with carbon, and filtered through a bed of celite. The solvent is evaporated and the resulting tan solid recrystallized from ethyl acetate/hexane, m.p. 166-169°C. (Found: C, 78.68; H, 6.39; N, 10.60. $C_{26}H_{25}N_3O$) requires: C, 78.96; H, 6.37; N, 10.62).

## Example 16

2-Methyl-3-(2-naphthalenyl)-5-(2-trans-phenylethenyl)-3-(1H-1,2,4-triazol-1-ylmethyl)isoxazolidine ($R^3$ = $CH=CHC_6H_5$, X = N)

A mixture of 2-(1H-1,2,4-triazol-1-yl)-N-methyl-1-(2-naphthalenyl)ethanimine N-oxide (15.0 g, 0.056 mol) and trans-1-phenyl-1,3-butadiene (11.0 g, 0.084 mol) in 500 ml toluene is heated to 95-105°C for 48 hours, then cooled and evaporated to dryness. The resulting oil is decolorized with carbon and purified by flash chromatography on silica gel (eluent comprised of 90% ethyl acetate and 10% hexane by volume) giving two diastereomers. Isomer A (5.66 g) has a melting point of 80-84°C (ethyl acetate-pentane). Isomer B (1.64 g) has a melting point 157-160°C.

## Example 17

5-Substituted-3-(2-naphthalenyl)-3-(1H-imidazol-1-ylmethyl)-2-methylisoxazolidines

By following essentially the same procedures as described for Example 14 and replacing p-methoxyphenyl allyl ether by

(a) allyl 4-methylphenyl sulfide, or

(b) allyl phenyl sulfide,

the corresponding 5-substituted-3-(2-naphthalenyl)-3-(1H-imidazol-1-ylmethyl)-2-methylisoxazolidines can be prepared.

For example,
5-{[(4-methylphenyl)thio]methyl}-3-(2-naphthalenyl)-3-(1H-imidazol-1-ylmethyl)-2-methylisoxazoldine,
5-[(phenylthio)methyl]-3-(2-naphthalenyl)-3-(1H-imidazol-1-ylmethyl)-2-methylisoxazolidine.

## Example 18

5-Substituted-3-(2-naphthalenyl)-3-(1H-1,2,4-triazol-1-ylmethyl)-2-methylisoxazolidines (X = N)

By following essentially the same procedures as described for Example 16 and replacing trans-1-phenyl-1,3-butadiene by

(a) 4-chlorostyrene, or

(b) allyl 4-methylphenyl sulfide, or

(c) allyl phenyl ether, or

(d) allyl 4-methoxyphenyl ether, or

(e) allyl 4-chlorophenyl ether, or

(f) 1-octene,
the corresponding 5-substituted-3-(2-naphthalenyl)-3-(1H-1,2,4-triazol-1-ylmethyl)-2-methylisoxazolidines can be prepared.

For example,
2-methyl-3-(2-naphthalenyl)-5-(4-chlorophenyl)-3-(1H-1,2, 4-triazol-1-ylmethyl)isoxazolidine,
5-[(phenylthio)methyl]-2-methyl-3-(2-naphthalenyl)-3-(1H-1,2,4-triazol-1-ylmethyl)isoxazolidine,
2-methyl-3-(2-naphthalenyl)-5-(phenoxymethyl)-3-(1H-1,2,4-triazol-1-ylmethyl)isoxazolidine,
5-[(4-chlorophenoxy)methyl]-2-methyl-3-(2-naphthalenyl)-3-(1H-1,2,4,-triazol-1-ylmethyl)isoxazolidine,
5-[(4-methoxyphenoxy)methyl]-2-methyl-3-(2-naphthalenyl)-3-(1H-1,2,4-triazol-1-ylmethyl)isoxazolidine,
5-hexyl-2-methyl-3-(2-naphthalenyl)-3-(1H-1,2,4-triazol-1-ylmethyl)isoxazolidine.

Salts of the compounds of the invention can be prepared as known in the art, for example, by dissolving the compound in a 10:1 by volume mixture of ethanol and aqueous acid such as HCl or $HNO_3$, evaporating the solvent, and then recrystallizing the crude salt, for example, from methanol-ether, 1:3 by volume in the case of HCl salts, and ethanol in the case of $HNO_3$ salts.

## Claims

1. A compound of the formula

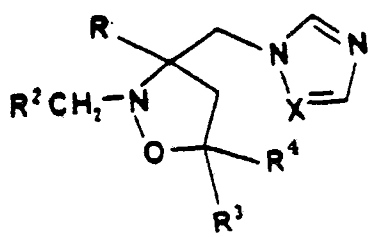

and the pharmaceutically acceptable acid addition salts thereof, in the form of their enantiomers or mixtures of their enantiomers including diastereomeric pairs of such enantiomers,
wherein X is selected from CH or N and wherein R, $R^2$, $R^3$ and $R^4$ are selected from the following combinations;

(a) R is,

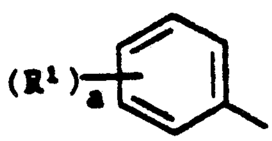

wherein a = 1 or 2, and
$R^1$ is selected from hydrogen, halogen, lower alkyl, lower alkoxy groups and combinations thereof, provided that the ortho position is hydrogen,
$R^2$ is,

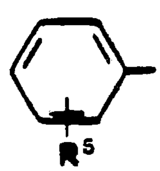

wherein $R^5$ is selected from hydrogen and one or more halogen, lower alkyl and lower alkoxy groups and combinations thereof,
$R^3$ is selected from phenyl, substituted phenyl, styryl, substituted styryl, substituted phenoxymethyl and substituted phenylthiomethyl groups wherein the substituents at the phenyl rings are selected from one or

more halogen, lower alkoxy and lower alkyl groups and combinations thereof, and
$R^4$ is H,

(b) R is defined as in (a) above,
$R^2$ is H, and
$R^3$ is selected from lower alkyl, phenyl, substituted phenyl and alkoxycarbonyl groups and
$R^4$ is selected from lower alkyl, phenyl and substituted phenyl groups, wherein the phenyl substituents are selected from halogen, lower alkyl, and lower alkoxy groups and combinations thereof, and

(c) R is naphthyl,
$R^2$ and $R^4$ are H, and
$R^3$ is selected from phenyl, substituted phenyl, phenoxymethyl, substituted phenoxymethyl, (phenylthio)-methyl, substituted (phenylthio)methyl, styryl and $C_2$ to $C_{18}$ alkyl, wherein the substituents on the substituted phenyl rings are selected from one or more of halogen, lower alkyl, lower alkoxy groups and combinations thereof.

2. A compound according to claim 1 wherein:
R is,

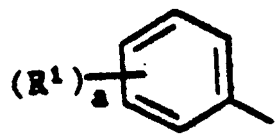

wherein a = 1 or 2, and
$R^1$ is selected from hydrogen, halogen, lower alkyl, lower alkoxy groups and combinations thereof, provided that the ortho position is hydrogen,
$R^2$ is,

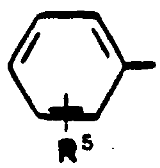

wherein $R^5$ is selected from hydrogen and one or more halogen, lower alkyl and lower alkoxy groups and combinations thereof,
$R^3$ is selected from phenyl, substituted phenyl, styryl, substituted styryl, substituted phenoxymethyl and substituted phenylthiomethyl groups wherein the substituents at the phenyl rings are selected from one or more halogen, lower alkoxy and lower alkyl groups and combinations thereof, and
$R^4$ is H.

3. A compound according to claim 1 wherein:
R is,

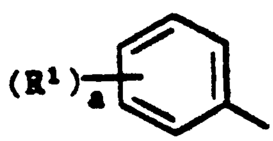

wherein a = 1 or 2, and
$R^1$ is selected from hydrogen, halogen, lower alkyl, lower alkoxy groups and combinations thereof, provided that the ortho position is hydrogen,
$R^2$ is H, and
$R^3$ is selected from lower alkyl, phenyl, substituted phenyl and alkoxycarbonyl groups and
$R^4$ is selected from lower alkyl, phenyl and substituted phenyl groups, wherein the phenyl substituents are selected from halogen, lower alkyl, and lower alkoxy groups and combinations thereof.

4. A compound according to claim 1 wherein:
R is naphthyl,
$R^2$ and $R^4$ are H, and
$R^3$ is selected from phenyl, substituted phenyl, phenoxymethyl, substituted phenoxymethyl, (phenylthio)-methyl, substituted (phenylthio)methyl, styryl and $C_2$ to $C_{18}$ alkyl, wherein the substituents on the substituted phenyl rings are selected from one or more of halogen, lower alkyl, lower alkoxy groups and combinations thereof.

5. A compound accoridng to claim 2 wherein the compound is 5-[(4-chlorophenoxy)methyl]-3-(1H-imidazol-1-ylmethyl)-3-phenyl-2-(phenylmethyl)isoxazolidine.

6. A compound according to claim 3 wherein the compound is 5-(4-chlorophenyl)-2,5-dimethyl-3-(1H-imidazol-1-ylmethyl)-3-phenylisoxazolidine.

7. A compound according to claim 4 wherein the compound is 3-(1H-imidazol-1-ylmethyl)-2-methyl-3-(2-naphthalenyl)-5-phenylisoxazolidine.

Claims for the following Contracting State: ES

1. A process for preparation of compounds of the formula:

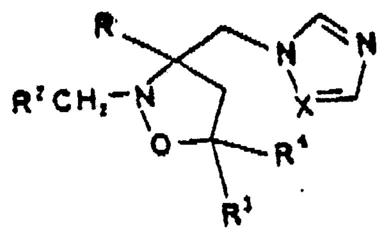

wherein x is selected from CH or N and wherein R, $R^2$, $R^4$ are selected from the following combinations

a) R is

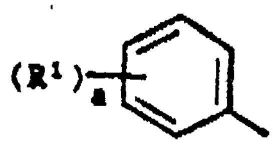

wherein a is 1 or 2, and $R^1$ is selected from hydrogen, halogen, lower alkyl, lower alkoxy groups and combinations thereof, provided that the ortho position is hydrogen,
$R^2$ is,

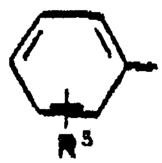

wherein $R^5$ is selected from hydrogen and one or more halogen, lower alkyl and lower alkoxy groups and combinations thereof,
$R^3$ is selected from phenyl, substituted phenyl, styryl, substituted styryl, substituted phenoxymethyl and substituted phenylthiomethyl groups wherein the substituents at the phenyl rings are selected from one or more halogen, lower alkoxy and lower alkyl and combinations thereof, and $R^4$ is H,

b) R is defined as in (a) above,
$R^2$ is H and
$R^3$ is selected from lower alkyl, phenyl, substituted phenyl and alkoxy carbonyl groups and
$R^4$ is selected from lower alkyl, phenyl and substituted phenyl, wherein the phenyl substituents are selected from halogen, lower alkyl and lower alkoxy groups and combinations thereof, and

c) R is naphthyl,
$R^2$ and $R^4$ are H, and $R^3$ is selected from phenyl, substituted phenyl, phenoxymethyl, substituted phenoxymethyl, (phenylthio)methyl, substituted (phenylthio)methyl, styryl and $C_2$ to $C_{18}$ alkyl wherein the substituents on the substituted phenyl rings are selected from one or more of halogen, lower alkyl, lower alkoxy groups and combinations thereof, characterized by: treating a nitrone of the formula

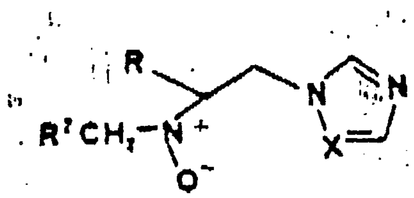

wherein x is as defined hereinabove R and $R^2$ are as defined hereinabove in a), b) or c) respectively with an alkene appropriately selected from the group consisting of
a')

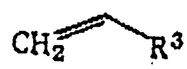

wherein $R^3$ is as defined hereinabove under a,
b')

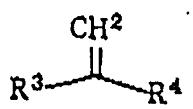

wherein $R^3$ and $R^4$ are as defined under b) above, and
c')

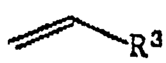

wherein $R^3$ is as defined in c) above.

2. A process as defined in claim 1 wherein:
R is,

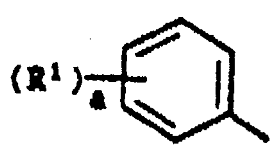

wherein a = 1 or 2, and
$R^1$ is selected from hydrogen, halogen, lower alkyl, lower alkoxy groups and combinations thereof, provided that the ortho position is hydrogen,
$R^2$ is,

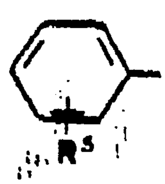

wherein $R^5$ is selected from hydrogen and one or more halogen, lower alkyl and lower alkoxy groups and combinations thereof,

$R^3$ is selected from phenyl, substituted phenyl, styryl, substituted styryl, substituted phenoxymethyl and substituted (phenylthio)methyl groups wherein the substituents at the phenyl rings are selected from one or more halogen, lower alkoxy and lower alkyl groups and combinations thereof, and $R^4$ is H.

3. A process as defined in claim 1 wherein:
R is,

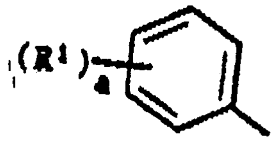

wherein a = 1 or 2, and
$R^1$ is selected from hydrogen, halogen, lower alkyl, lower alkoxy groups and combinations thereof, provided that the ortho position is hydrogen,
$R^2$ is H, and
$R^3$ is selected from lower alkyl, phenyl, substituted phenyl and alkoxycarbonyl groups and $R^4$ is selected from lower alkyl, phenyl and substituted phenyl groups, wherein the phenyl substituents are selected from halogen, lower alkyl, and lower alkoxy groups and combinations thereof.

4. A process as defined in claim 1 wherein:
R is naphthyl,
$R^2$ and $R^4$ are H, and
$R^3$ is selected from phenyl, substituted phenyl, phenoxymethyl, substituted phenoxymethyl, (phenylthio)-methyl, substituted (phenylthio)methyl, styryl and $C_2$ to $C_{18}$ alkyl, wherein the substituents on the substituted phenyl rings are selected from one or more of halogen, lower alkyl, lower alkoxy groups and combinations thereof.

5. A process as defined in claim 2 wherein $R^1$ and $R^5$ are hydrogen, and $R^3$ is $-CH_2OC_6H_4Cl$-(4).

6. A process according to claim 3 wherein $R^1$ is hydrogen, $R^3$ is methyl and $R^4$ is $-C_6H_4$-Cl-(4).

7. A process as defined in claim 4 wherein $R^3$ is phenyl.

European Patent Office

# EUROPEAN SEARCH REPORT

Application Number

EP 88 11 6061

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| A | EP-A-0 171 137 (UNIROYAL)<br>--- | | C 07 D 413/06<br>A 61 K 31/42 |
| A | EP-A-0 192 060 (NIHON TOKUSHU NOYAKU SEIZO)<br>--- | | |
| P,A | EP-A-0 257 391 (PENNWALT CORP.)<br>--- | | |
| P,A | EP-A-0 257 351 (PENNWALT CORP.)<br>----- | | |
| | | | TECHNICAL FIELDS SEARCHED (Int. Cl.4) |
| | | | C 07 D 413/06<br>A 61 K 31/42 |

The present search report has been drawn up for all claims

| Place of search | Date of completion of the search | Examiner |
| --- | --- | --- |
| THE HAGUE | 15-12-1988 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document